# EUROPEAN PATENT APPLICATION

(11) **EP 1 462 011 A1**
(43) Date of publication of application: **29.09.2004**
(21) Application number: 03251833.4
(22) Date of filing: 24.03.2003
(51) Int. Cl.: A23L 1/307, A23L 1/09, A23L 1/236, A23L 2/60, A61K 31/7016

(54) **Comestibles containing Isomaltulose and Trehalose for sustained carbohydrate energy release and reduced glycemic/insulinimic responses**

(71) Applicant: CERESTAR HOLDING B.V., 4551 LA Sas Van Gent (NL)
(72) Inventor: Brouns, Fredericus J., 6301 AW Sibbe-Valkenburg (NL); Simon, Jacques G., 92140 Clamart (FR); Bonnet, Didier, 1180 Uccle (BE)
(74) Representative: Wilkinson, Stephen John

(57) **Abstract**

The current invention relates to a composition comprising as sustained carbohydrate energy release source a mixture of isomaltulose and trehalose, and optionally a carbohydrate selected from the group consisting of fructose, fructose syrups, sucrose, invert sugar, and mixtures thereof. This composition is applied in solid, semi-solid and liquid comestibles.

## Description

### Technical Field

The present invention relates to a composition of isomaltulose and trehalose and optionally a carbohydrate selected from the group consisting of fructose, fructose syrups, sucrose, invert sugar, and mixtures thereof for sustained carbohydrate energy release in liquid, semi-solid and solid comestibles.

### Background of the invention

There are a number of liquid, semi-solid and solid products currently applied for providing energy to the body.

A lot of liquid compositions or diluted mixtures are on the market by the name of 'Activity drinks', 'Sports drinks', 'Energy drinks' or 'Nutrient drinks'. These drinks are reported to meet requirements with respect to the use and/or loss of carbohydrates, electrolytes, vitamins, electrolytes, amino acids, and other important nutrients which occurs during heavy exercise.

JP01-060360A relates to an isotonic drink which is containing palatinose (= isomaltulose) as main carbohydrates.

JP63-112963A relates to food and drink which is containing palatinose as a sweetener, and/or excipient, and/or extender.

EP 0 882 408 relates to a method for improving the aftertaste of sucrose without lowering the sweetening power of sucrose, and which comprises incorporating trehalose into sucrose in an amount of 2 to 12% to the sucrose.

JP2001069941A2 (Abstract) relates to a composition which retains a sugar-like flavor by coexistence of fructose and trehalose in a ratio of 1: (0.4 to 1).

JP2000262216A2 (Abstract) relates to a sweetener containing coffee beverage comprising trehalose in an amount corresponding to 10 -30% of the sweetener.

JP9173017A2 (Abstract) relates to a beverage or food containing trehalose for improvement of physical strength.

There is a further need for having compositions suitable for sustained carbohydrate energy release.

The current invention provides such a composition and products comprising this composition.

### Summary of invention

The current invention relates to a composition comprising as sustained carbohydrate energy release source a mixture of isomaltulose and trehalose. In a preferred embodiment such a composition is comprising a mixture of isomaltulose and trehalose in a ratio from 10:90 to 90:10.

The current invention further relates to a composition comprising as sustained carbohydrate energy release source a mixture of isomaltulose and trehalose and further a carbohydrate (A) selected from the group consisting of fructose, fructose syrups, sucrose, invert sugar, and mixtures thereof. In a preferred embodiment the ratio of said carbohydrate (A) to mixture of isomaltulose and trehalose is from 30:70 to 80:20.

The current invention further relates to a solid, semi-solid or liquid comestible wherein (based on dry matter of comestible) at least 5% of composition containing a mixture of isomaltulose and trehalose is present, or at least 5% of a composition containing a mixture of isomaltulose and trehalose and carbohydrate (A) selected from the group consisting of fructose, fructose syrups, sucrose, invert sugar, and mixtures thereof.

The current invention further relates to a comestible containing the composition wherein a mixture of isomaltulose and trehalose and said carbohydrate (A) are present in a ratio of carbohydrate (A) to mixture of from 30:70 to 80:20.

Preferably these compositions represents more than 10% of dry substance of the comestible.

Said comestible is selected from the group consisting of tablets, bars, confectionery, beverages, beverage concentrates, gels, drink powders, diabetic food, baby food, infant food, dietetic food, slimming food, food for special dietary needs and medical food.

Furthermore, the current invention relates to a beverage selected from the group consisting of hypotonic drinks, soft drinks, sports drinks, hypertonic drinks, energy drinks and isotonic drinks. The beverage further can comprise other carbohydrates, proteins, peptides, amino acids, antioxidants, fats, vitamins, trace elements, electrolytes, intense sweeteners, edible acids, flavors and/or mixtures thereof. These carbohydrates are selected from the group consisting of monosaccharides, disaccharides, gelling starches, starch hydrolysates, dextrins, polyols and mixtures thereof.

The dry substance of said carbohydrates in the beverage is comprising at least 50% of a composition comprising as sustained carbohydrate energy release source a mixture of isomaltulose and trehalose or at least 50% of a composition containing a mixture of isomaltulose and trehalose and carbohydrate (A) selected from the group consisting of fructose, fructose syrups, sucrose, invert sugar, and mixtures thereof.

The current invention further relates to beverages wherein the dry substance of the carbohydrates is containing at least 50% of a composition containing a mixture of isomaltulose and trehalose and carbohydrate (A) selected from the group consisting of fructose, fructose syrups, sucrose, invert sugar, and mixtures thereof, and wherein the ratio of carbohydrate (A) to mixture is from 30:70 to 80:20.

Furthermore, the current invention further relates to the use of a mixture of isomaltulose and trehalose for sustained carbohydrate energy release in athletics food, dietetic food, food for special dietary needs, diabetics food, baby food, infant food, food for elderly, and medical food.

In addition, the current invention relates to the use of compositions comprising a mixture of isomaltulose and trehalose and which is further comprising a carbohydrate (A) selected from the group consisting of fructose, fructose syrups, sucrose, invert sugar, and mixtures thereof for sustained carbohydrate energy release in athletics food, dietetic food, food for special dietary needs, slimming food, diabetics food, baby food, infant food, food for elderly, and medical food.

The current invention further relates to the use of a mixture of isomaltulose and trehalose to modify perception of satiety or hunger.

It further relates to to the use of compositions comprising a mixture of isomaltulose and trehalose and which is further comprising a carbohydrate (A) selected from the group consisting of fructose, fructose syrups, sucrose, invert sugar, and mixtures thereof to modify perception of satiety or hunger.

### Description of the figures

- Figure 1:: Typical curve for glucose response in blood of a mixture of isomaltulose and trehalose in a ratio of 50:50.
- Figure 2:: Typical curve for insulin response in blood of a mixture of isomaltulose and trehalose in a ratio of 50:50.

### Detailed invention

The current invention relates to a composition comprising as sustained carbohydrate energy release source a mixture of isomaltulose and trehalose.

Isomaltulose or 6-O-α-D-glucopyranosyl-D-fructofuranose is synthesised from sucrose by the action of an enzyme present in bacterial strains like *Protaminobacter* rubrum, *Erwinia* rhapontici and *Serratia* plymuthica.

Trehalose (α-D-glucopyranosyl-α-D-glucopyranoside) has been known as non-reducing saccharide composed of glucose units.

In a preferred embodiment such a composition is comprising a mixture of isomaltulose and trehalose in a ratio from 10:90 to 90:10.

The current invention further relates to a composition comprising as sustained carbohydrate energy release source a mixture of isomaltulose and trehalose and further a carbohydrate (A) selected from the group consisting of fructose, fructose syrups, sucrose, invert sugar, and mixtures thereof. In a preferred embodiment the ratio of said carbohydrate (A) to mixture of isomaltulose and trehalose is from 30:70 to 80:20.

The composition is particular useful for providing carbohydrate energy over a long period, while the composition is digestible, and absorbable.

Fructose syrups cover all syrups containing on dry substance from 42 to 100% fructose. An example of a fructose syrup can be high fructose corn syrup which is containing from 42-55% fructose.

The current invention further relates to a solid, semi-solid or liquid comestible wherein (based on dry matter of comestible) at least 5% of composition containing a mixture of isomaltulose and trehalose is present, or at least 5% of a composition containing a mixture of isomaltulose and trehalose and carbohydrate (A) selected from the group consisting of fructose, fructose syrups, sucrose, invert sugar, and mixtures thereof.

The current invention further relates to a comestible containing the composition wherein a mixture of isomaltulose and trehalose and said carbohydrate (A) are present in a ratio of carbohydrate (A) to mixture of from 30:70 to 80:20.

Preferably these compositions represents more than 10% of dry substance of the comestible.

Said comestible is selected from the group consisting of tablets, confectionery, bars, beverages, beverage concentrates, gels, drink powders, diabetics food, baby food, infant food, dietetic food, slimming food, food for special dietary needs, and medical food.

Tablets can be based solely upon the composition of isomaltulose and trehalose or a composition of said mixture and carbohydrate (A) selected from the group consisting of fructose, fructose syrups, sucrose, invert sugar, and mixtures thereof, for obtaining sustained carbohydrate energy release.

The diabetic food, baby food, infant food, dietetic food, slimming food, food for special dietary needs refer to any type of food suitable for diabetics, babies, infants and people needing a special dietetic formulation and any one who can benefit from the presence of a sustained carbohydrate energy release source, and those who can benefit from a modified perception of satiety or hunger.

Medical food refers to any liquid, semi-solid or liquid comestible which is given to people in medical need for having access to extra sustained carbohydrate energy source, e.g. people with heavy bums and/or scalds.

The beverage can be any drinkable solution including iced tea, and fruit juices, vegetable based juices, lemonades, cordials, nut based drinks, cocoa based drinks, dairy products such as milk, whey, yogurts and drinks based on them.

Beverage concentrate refers to a concentrate that is either in liquid form or in essentially dry mixture form. The liquid concentrate can be in the form of a relatively thick, syrupy liquid. The essentially dry mixture can be in the form of either a powder or a tablet. The beverage concentrate is usually formulated to provide a drinkable beverage composition or a final beverage when constituted or diluted with water, either carbonated or non-carbonated.

Drink powders are suitable for constituting with water, carbonated or non-carbonated, a final beverage for oral administration of sustained energy release source of current invention.

Furthermore, the current invention relates to a beverage selected from the group consisting of hypotonic drinks, soft drinks, sports drinks, hypertonic drinks, energy drinks and isotonic drinks.

A specific example of a hypotonic drink is a rehydration drink.

In general, the beverage can further be characterized in having an osmolality of from 50 to 800 mOs/L, preferably from 150 to 600 mOs/L, more preferably from 200 to 400 mOs/L.

An isotonic drink is typically characterized by an osmolality of from 270 - 330 mOs/L.

The beverage further can comprise other carbohydrates, proteins, peptides, amino acids, antioxidants, fats, vitamins, trace elements, electrolytes, intense sweeteners, edible acids, flavors and/or mixtures thereof.

The carbohydrates are selected from the group consisting of monosaccharides, disaccharides, gelling starches, starch hydrolysates, dextrins, polyols and mixtures thereof, whereby these carbohydrates are different from isomaltulose, trehalose and carbohydrate (A) as mentioned in the composition of current invention.

The monosaccharides include tetroses, pentoses, hexoses and ketohexoses.

Starch hydrolysates are produced by the controlled acid or enzymatic hydrolysis of starch and can be subdivided into two specific categories, maltodextrins and glucose syrups and are characterizedby DE number (dextrose equivalent). In fact, DE number is a measurement of the percentage of reducing sugars present in the syrup and calculated as dextrose on a dry weight basis. Maltodextrins have a DE number up to 20 whereas glucose syrups have an DE number greater than 20.

Dextrins are prepared according to the dextrinisation method. Dextrinisation is a heat treatment of dry starch in presence or absence of acid.

Gelly starches may include emulsified starches such as starch n-octenyl succinate.

The dry substance of said carbohydrates in the beverage is comprising at least 50% of a composition comprising as sustained carbohydrate energy release source a mixture of isomaltulose and trehalose or at least 50% of a composition containing a mixture of isomaltulose and trehalose and carbohydrate (A) selected from the group consisting of fructose, fructose syrups, sucrose, invert sugar, and mixtures thereof.

The current invention further relates to beverages wherein the dry substance of the carbohydrates is containing at least 50% of a composition containing a mixture of isomaltulose and trehalose and carbohydrate (A) selected from the group consisting of fructose, fructose syrups, sucrose, invert sugar, and mixtures thereof, and wherein the ratio of carbohydrate (A) to mixture is from 30:70 to 80:20.

In fact, it is further possible having beverages wherein the dry matter content of the carbohydrates is based 100% upon the composition of the current invention, i.e composition containing a mixture of isomaltulose and trehalose or composition containing said mixture and a carbohydrate (A) selected from the group consisting of fructose, fructose syrups, sucrose, invert sugar, and mixtures thereof.

Among the major physiological electrolytes are sodium, potassium, chloride, calcium, and magnesium. Further trace elements can be included such as chromium, copper, selenium, iron, manganese, molybdenym, zinc and mixtures thereof.

Among the vitamins one can range vitamin A, vitamin C, vitamin E and/or vitamin B₁₂.

The edible acids can be selected from phosphoric acid, citric acid, malic acid, succinic acid, adipic acid, gluconic acid, tartaric acid, fumaric acid and mixtures thereof. Preferably the pH range of the beverage is from about 2 to about 6.5.

The intense sweeteners include, but are not limited to, saccharin, cyclamates, acetosulfam, aspartame and the like.

The flavors are selected from fruit flavors, botanical flavors and mixtures thereof. Preferred flavors are cola flavor, grape flavor, cherry flavor, apple flavor and citrus flavors such as orange flavor, lemon flavor, lime flavor, fruit punch and mixtures thereof. The amount of flavor depends upon the flavor or flavors selected, the flavor impression desired and the form of flavor used.

If desired, coloring agents can also be added. Any soluble coloring agent approved for food use can be utilized for the current invention.

When desired, preservatives such as potassium sorbate and sodium benzoate can be added.

Gums, emulsifiers and oils can also be added in the beverage for texture and opacity purposes. Typical ingredients include guar gum, xanthan gum, alginates, carboxymethylcellulose, mono-di- glycerides, lecithin, gelling starches, pectin, pulp, cotton seed oil and vegetable oil.

The beverage may be prepared by mixing together all of the ingredients. The mixture is then dissolved in water and agitated until all the ingredients are dissolved. Dissolution may occur at ambient temperature but it may be necessary for the solution to be heated to temperature between 50-100°C to get all the ingredients into solution. After the mixture having been adjusted to a desired pH, the beverage may be bottled, capped, and eventually pasteurized at about 75°C for about 20 minutes, or the beverage may be before bottling continuously pasteurized for a few minutes.

One way to prepare the concentrate of the beverage would be to start with less than the required volume of the liquid solvent that is used to prepare the drinkable beverage. Another way would be to partially dehydrate the finally prepared drinkable beverage to remove only a portion of the liquid solvent and any other volatile liquid present.

Carbon dioxide can be introduced either into the water to be mixed with the beverage concentrate or into the drinkable beverage to achieve carbonation. The carbonated beverage can then be stored in a container, such as a bottle or a can and then be sealed.

The current invention relates to the use of a mixture of isomaltulose and trehalose or to the use of compositions comprising a mixture of isomaltulose and trehalose and which is further comprising a carbohydrate (A) selected from the group consisting of fructose, fructose syrups, sucrose, invert sugar, and mixtures thereof.

Furthermore, the current invention further relates to the use of a mixture of isomaltulose and trehalose for sustained carbohydrate energy release in athletics food, dietetic food, food for special dietary needs, slimming food, diabetics food, baby food, infant food, food for elderly, and medical food.

In addition, the current invention relates to the use of compositions comprising a mixture of isomaltulose and trehalose and which is further comprising a carbohydrate (A) selected from the group consisting of fructose, fructose syrups, sucrose, invert sugar, and mixtures thereof for sustained carbohydrate energy release in athletics food, dietetic food, food for special dietary needs, slimming food, diabetics food, baby food, infant food, food for elderly, and medical food.

Furthermore, the current invention relates to the use of a mixture of isomaltulose and trehalose or compositions comprising a mixture of isomaltulose and trehalose and which is further comprising a carbohydrate (A) selected from the group consisting of fructose, fructose syrups, sucrose, invert sugar, and mixtures thereof to modify perception of satiety or hunger.

The current invention has the following advantages:
- The composition comprising a mixture of isomaltulose and trehalose is a suitable source of sustained carbohydrate energy release.
- The composition comprising a mixture of isomaltulose and trehalose and a carbohydrate (A) selected from the group consisting of fructose, fructose syrups, sucrose, invert sugar, and mixtures thereof, is particularly useful as a source of sustained carbohydrate energy release.
- Said composition is digestible, and absorbable.
- The composition can be applied in solid, semi-solid and liquid comestibles.
- The comestible is suitable for athletics, diabetics, babies, infants, elderly people and those requiring a special diet in respect of sustained carbohydrate energy release.
- The comestible is suitable for people following a slimming diet due to the modified perception of satiety or hunger.

The current invention is illustrated by way of the following examples.

### Example 1

The basic syrup was prepared with the following ingredients:
152 g isomaltulose
160 g trehalose
291 g fructose
5 ml sodium benzoate 10% (w/v)
3 ml phosphoric acid 85%
15 g cola flavor Wild (nr 35103000170000)
carbonated water was added for obtaining 1 liter basic syrup.
42 ml of this basic syrup was placed in a bottle and further diluted with carbonated water to a final volume of 210 ml.
The taste was evaluated with a taste panel.
A good cola perception was found, comparable to a standard drink prepared with 534 g sucrose.

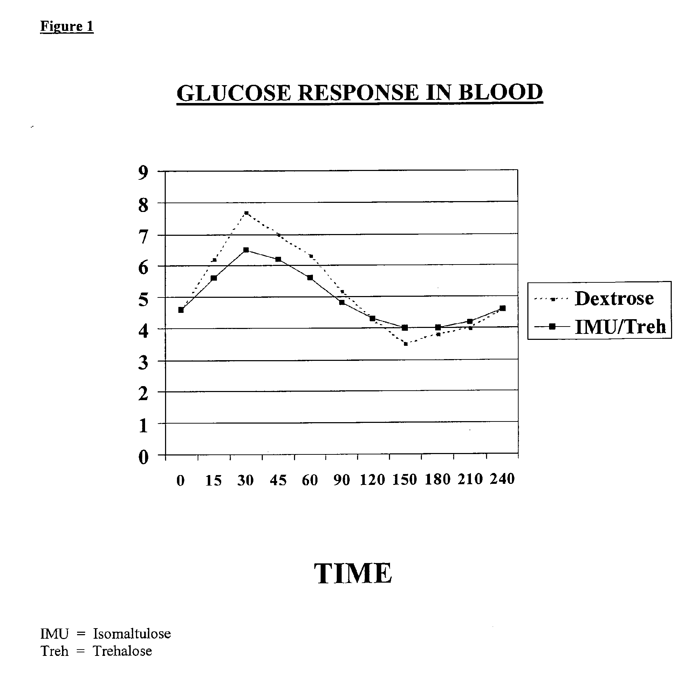

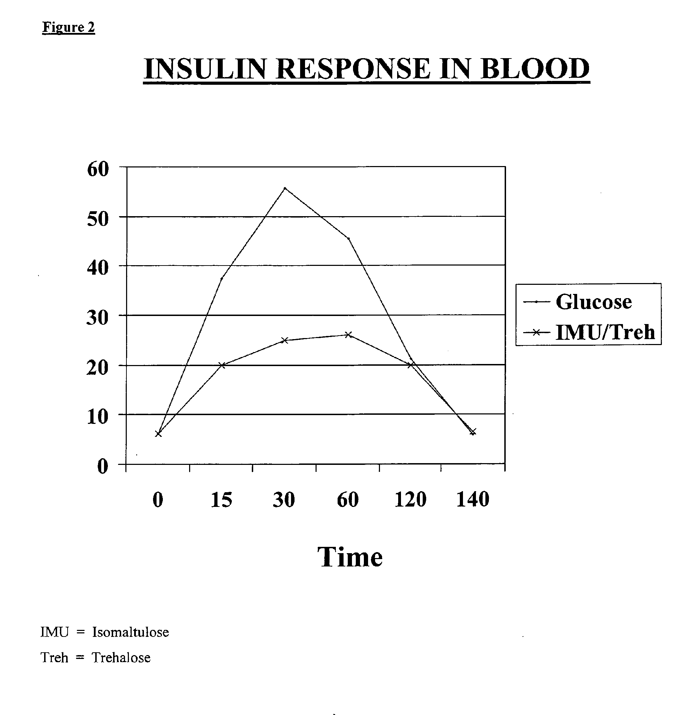

The glucose and insulin response of mixtures comprising isomaltulose and trehalose in a ratio of 50:50 are displayed in Figure 1 and 2.

## Claims

1. Composition comprising as sustained carbohydrate energy release source a mixture of isomaltulose and trehalose.

2. A composition according to claim 1 **characterized in that** said composition is comprising said mixture of isomaltulose and trehalose in a ratio from 10:90 to 90:10.

3. A composition according to claim 1 or 2 **characterized in that** said composition is further comprising a carbohydrate (A) selected from the group consisting of fructose, fructose syrups, sucrose, invert sugar and mixtures thereof.

4. A composition according to claim 3 **characterized in that** in said composition the ratio of carbohydrate to mixture of isomaltulose and trehalose is from 30:70 to 80:20.

5. A solid, semi-solid or liquid comestible **characterized in that** dry matter of comestible is comprising at least 5% of a composition containing a mixture of isomaltulose and trehalose or at least 5% of a composition containing a mixture of isomaltulose and trehalose and carbohydrate (A) selected from the group consisting of fructose, fructose syrups, sucrose, invert sugar, and mixtures thereof

6. A comestible according to claim 5 **characterized in that** in said composition containing a mixture of isomaltulose and trehalose and said carbohydrate (A) the ratio of carbohydrate (A) to mixture is from 30:70 to 80:20.

7. A comestible according to claim 5 **characterized in that** dry matter of comestible is comprising more than 10% of a composition containing a mixture of isomaltulose and trehalose or more than 10% of a composition containing a mixture of isomaltulose and trehalose and carbohydrate (A) selected from the group consisting of fructose, fructose syrups, sucrose, invert sugar, and mixtures thereof.

8. A comestible according to anyone of claims 5 to 7 **characterized in that** said comestible is selected from the group consisting of tablets, bars, confectionery, beverages, beverage concentrates, gels, drink powders, diabetic food, baby food, infant food, dietetic food, slimming food, food for special dietary needs, and medical food.

9. A beverage according to claim 8 **characterized in that** said beverage is selected from the group consisting of hypotonic drinks, soft drinks, sports drinks, hypertonic drinks, energy drinks, and isotonic drinks.

10. A beverage according to claim 9 **characterized in that** it is further comprising carbohydrates, proteins, peptides, amino acids, antioxidants, fats, vitamins, trace elements, electrolytes, intense sweeteners, edible acids, flavors and/or mixtures thereof.

11. A beverage according to claim 10 **characterized in that** said carbohydrates are selected from the group consisting of monosaccharides, disaccharides, gelling starches, starch hydrolysates, dextrins, polyols and mixtures thereof.

12. A beverage according to claim 11 **characterized in that** dry substance of said carbohydrates in said beverage is comprising at least 50% of a composition containing a mixture of isomaltulose and trehalose or at least 50% of a composition containing a mixture of isomaltulose and trehalose and carbohydrate (A) selected from the group consisting of fructose, fructose syrups, sucrose, invert sugar, and mixtures thereof.

13. A beverage according to claim 12 **characterized in that** in said composition containing a mixture of isomaltulose and trehalose and said carbohydrate (A) the ratio of carbohydrate (A) to mixture is from 30:70 to 80:20.

14. Use of a composition comprising isomaltulose and trehalose for sustained carbohydrate energy release in athletics food, dietetic food, food for special dietary needs, slimming food, diabetics food, baby food, infant food and food for elderly, and medical food.

15. Use according to claim 14 **characterized in that** said composition is further comprising carbohydrate (A) selected from the group consisting of fructose, fructose syrups, sucrose, invert sugar, and mixtures thereof.

16. Use of a composition comprising isomaltulose and trehalose to modify perception of satiety or hunger.

17. Use according to claim 16 **characterized in that** said composition is further comprising carbohydrate (A) selected from the group consisting of fructose, fructose syrups, sucrose, invert sugar, and mixtures thereof.
